# EUROPEAN PATENT SPECIFICATION

(11) **EP 3 079 467 B1**
(45) Date of publication and mention of the grant of the patent: **07.08.2019**
(21) Application number: 13798316.9
(22) Date of filing: 27.11.2013
(51) Int. Cl.: A01N 33/04, A01N 31/14, A01N 33/12, A61L 2/16, A01P 1/00, C11D 7/26, C11D 7/32

(54) **DISINFECTANT CLEANER COMPOSITION HAVING TUBERCULOCIDAL EFFICACY AND EFFICACY AGAINST SPECIFIC VIRUSES**
DESINFEKTIONSREINIGERZUSAMMENSETZUNG MIT TUBERKULOZIDER WIRKUNG UND WIRKSAMKEIT GEGEN SPEZIFISCHE VIREN
COMPOSITION DE NETTOYAGE DÉSINFECTANTE AYANT UNE EFFICACITÉ TUBERCULOCIDE ET UNE EFFICACITÉ CONTRE DES VIRUS SPÉCIFIQUES

(43) Date of publication of application: 19.10.2016
(73) Proprietor: Ecolab USA Inc., St. Paul, MN 55102 (US)
(72) Inventor: LOHRMANN, Elke, 51429 Bergisch Gladbach (DE); KURZAWE, Christoph, 41469 Neuss (DE); MEYER, Bernhard, 40822 Mettmann (DE)
(74) Representative: Michalski Hüttermann & Partner Patentanwälte mbB
(86) International application number: PCT/EP2013/074852
(87) International publication number: WO 2015/078496

(56) References cited:
- EP-A1- 0 343 605
- DE-A1- 19 526 481
- DE-A1-102006 006 765
- FR-A1- 2 383 671
- US-A- 4 797 420
- US-A- 4 983 635
- US-A1- 2012 225 948
- Ecolab: "Sicherheitsdatenblatt Gemäss Verordnung (EG) Nr. 1907/2006 (Reach), Anhang II (453/2010)_Europa, INcidin Pro", 18 September 2013 (2013-09-18), Ecolab pages 1-16,
- "Declaration from Jens Korr, European segment manager instrument reprocessing at ECOLAB", 30 January 2019 (2019-01-30)
- Declaration from Paulina Hauzer, senior regulatory affairs manager for product safety, hazard communication and transport complian: 30 January 2019 (2019-01-30)

## Description

### Field of the Invention

The present invention is directed to a disinfectant cleaner composition that is effective in for inactivating and/or reducing infectious agents, of mycobacteria, and/or Adenovirus and Simianvirus 40. In particular, the present invention relates to an N-alkyl dimethyl benzyl ammonium chloride disinfectant that typically does not have tuberculocidal efficacy, but in association with bis(3-aminopropyl) alkylamine and phenoxy ethanol, surprisingly it is very effective in inactivating of mycobacteria, and/or Adenovirus and Simianvirus 40.

### Background of the Invention

Microbes may often be present on many common objects and surfaces in everyday life. Microbes can include, for example, bacteria, fungi, spores, viruses, prions, microorganisms such as, e.g., Mycobacterium tuberculosis, listeria monocytogenas, escherichia coli, pseudomonas aeruginosa, salmonella typhimurium, salmonella enteritidis, Yersinia pestis, staphylococcus aureus, bacillus subtilis, enterobacter aerogenes, streptococcus faecalis, legionella pneumophila, vibrio parahaemolyticus, bacillus cereus, and other gram positive and gram negative bacteria. Several such microbes/ microorganisms, individually or in combination, can cause illness or other health problems, for example, when they come into contact with humans and/or animals, or when they are ingested along with food which has contacted them. These microbes present health hazards due to infection or contamination. When microorganisms are present on the surface of a substrate they can replicate rapidly to form colonies. The microbial colonies form a coating on the substrate surface which is known as a biofilm. Biofilms frequently consist of a number of different species of microorganisms which in turn can be snore difficult to eradicate and thus more hazardous to health than individual microorganisms. Some microorganisms also produce polysaccharide coatings, which makes them more difficult to destroy.

For example, hospitals and other medical facilities may have a particular need for sterile and/or uncontaminated surfaces, both in surgical and general medical areas as well as in convalescence facilities, where patient exposure may be significant and resistance to such microbes may be lowered.

Much time and effort can be spent, for example, on disinfecting and sterilizing medical instruments, testing devices. Often, such devices can be provided with disposable components or covers (e.g., disposable thermometer probes) to avoid cross-contamination between patients. Disposable needles are also commonly used. Such disposable materials involve increased costs and increased waste, as well as potential safety issues associated with their disposal.

The food-preparation and delivery industry is another area in which presence of microbes (e.g., Bacteria) can be problematic. Food preparation facilities, if contaminated with microbes, can lead to contamination of food which may cause health problems when ingested. For example, restaurants, food manufacturing plants, and even home kitchens can contain preparation surfaces, utensils, and equipment which may contaminate food that comes into contact with them. There may be, for example, a particular need for reducing a presence and spreading of microbes in meat packaging plants.

Public and private facilities such as, e.g., restrooms, may also contain surfaces which can harbor and spread microbes, leading to potential health problems. To address this issue, products such as antimicrobial soaps and air dryers for hands may be offered, as well as disposable paper towels. Nevertheless, microbes may still be harbored on such objects as faucet and toilet handles, door knobs, keys, dispenser levers.

In the transportation industry, including land, sea, air, and space vehicles, there may also be particular surfaces which can harbor and spread microbes, leading to potential health problems. For example, rental cars may benefit from disinfecting frequently touched surfaces. In particular, isolated environments such as, e.g., airplanes and submarines can also be safer if surfaces are disinfected.

Other common objects may benefit from antimicrobial treatments, which can inhibit or prevent spread of microorganisms between people and/or animals that come into contact with such objects. For example, musical instruments, such as harmonicas, flutes, clarinets, computer peripherals, communications equipment such as, e.g., telephones, pet accessories such as leashes and carriers, and/or other common household objects could benefit from antimicrobial surfaces.

U.S. Pat. No. 8,455,551 relates to a phenol-free broad spectrum disinfectant cleaner composition comprising an alkyl ammonium halogen, a solvent, an alkaline agent, a chelant, a nonionic surfactant coupler, and wetting and emulsifying surfactants that is effective in eradicating microorganisms such as Aspergillus niger and Aspergillus brasiiiensis. However, the phenol-free broad spectrum disinfectant cleaner composition is not effective in inactivating viruses such as Norovirus, Adenovirus and Polyomavirus. Further the described phenol-free broad spectrum disinfectant cleaner composition having no tuberculocidal efficacy.

EP 0 343 605 A1 refers to the use of N, N-Bis-(3-aminopropyl)-laurylamine as effective components in a liquid aldehyde-free composition active against tuberculosis.

DE 195 26 481 A1 refers to a powdered aldehyde and phenol free cleansing instrument disinfectants containing N, N-bis (3-aminopropyl) lauryl amine, a quaternary ammonium compound, a powdered complexing and a powdery, chemically neutral carrier substance.

FR 2 383 671 A1 refers to disinfectants effective against mycobacterium tuberculosis containing germicidal quaternary ammonium salt and an alkoxylated amine compound.

US 2012/225948 A1 refers to a broad spectrum disinfectant includes a quaternary ammonium halogen, an alkaline agent, a chelating agent, a nonionic surfactant coupler, at least one alkoxylated nonionic surfactant, and water or any aliphatic alcohol. The disinfectant composition is phenol-free, is effective in eradicating microorganisms such as various fungi, and is stable to gamma-irradiation.

US 4 797 420 A refers to a formulation effective against microorganisms including herpes simplex types 1 and 2 viruses, comprising an alkyl (58% C14, 28% C16, 14% C12) dimethyl benzyl ammonium chloride.

US 4 983 635 A refers to a quaternary ammonium compound with dual synergistic phenols is provided for disinfection and sanitization. This formula consists of dual chain quaternary ammonium (N-alkyldimethylethylbenzyl ammonium chloride N-alkyldimethylbenzyl ammonium chloride), ortho phenyl phenol, paratertiary amyl phenol, isopropyl alcohol, citric acid, and water.

Quaternary ammonium chloride-containing products (also known as "quats") have been used in hard surface disinfection for many years. As a broad-spectrum disinfectant, they have acceptable efficacy against some organisms (e.g. Staphylococcus aureus), but often fall short in efficacy against mycobacteria and viruses. Thus, other harsher disinfectant or sporicidal products, such as Sodium chlorite (NaClO₂), ozone, H₂O₂ and peracetic acids are often used when mycobactericidal and virucidal efficacy is required.

Compositions and procedures have been developed to reduce or prevent a presence of microbes on certain surfaces. More importantly, such compositions may often not provide a rapid and high level of microbial efficacy, in particular a sufficient virucidal efficacy which could reduce the risk of microbial contaminants. Further, peroxide or chlorite containing antimicrobial compositions may have a material destructive effect on surfaces.

One can see that there is a continuing need for improved antimicrobial cleaners that are material non-destructive and having a rapid and high level of tuberculocidal and mycobactericidal efficacy and a rapid and high level of efficacy against Norovirus, Adenovirus and Polyomavirus.

### Summary of the Invention

The present invention is directed in accordance with claim 1 to a disinfectant cleaner composition comprising:
a) at least one quaternary ammonium chloride of N-alkyl dimethyl benzyl ammonium chloride, wherein said alkyl has from 8 to 18 carbon atoms;
b) at least one bis(3-aminopropyl) alkylamine, wherein said alkyl has from 6 to 18 carbon atoms;
c) phenoxy ethanol; and
wherein the weight-% ratio of a) a quaternary ammonium chloride of N-alkyl dimethyl benzyl ammonium chloride, wherein said alkyl has from 8 to 18 carbon atoms to b) bis(3-aminopropyl) alkylamine, wherein said alkyl has from 6 to 18 carbon atoms, is in the range of > 0.1 : 1 to < 1 : 1.

It has been surprisingly found that a disinfectant cleaner composition comprising a) at least one quaternary ammonium chloride of N-alkyl dimethyl benzyl ammonium chloride, wherein said alkyl has from 8 to 18 carbon atoms; b)at least one bis(3-aminopropyl) alkylamine, wherein said alkyl has from 6 to 18 carbon atoms; c) phenoxy ethanol; and wherein the weight-% ratio of a) a quaternary ammonium chloride of N-alkyl dimethyl benzyl ammonium chloride, wherein said alkyl has from 8 to 18 carbon atoms to b) bis(3-aminopropyl) alkylamine, wherein said alkyl has from 6 to 18 carbon atoms, is in the range of > 0.1 : 1 to < 1 : 1, is material non-destructive and having a rapid and high level of mycobacteria, and/or Adenovirus and Simianvirus 40 efficacy.

According to one aspect of the invention the weight ratio of a) a quaternary ammonium chloride of N-alkyl dimethyl benzyl ammonium chloride, wherein said alkyl has from 8 to 18 carbon atoms, to b) bis(3-aminopropyl) alkylamine, wherein said alkyl has from 6 to 18 carbon atoms, preferably bis(3-aminopropyl) dodecylamine, can be in the range of ≥ 0.2 : 1 to ≤ 0.99 : 1, preferably of ≥ 0.3 : 1 to ≤ 0.95 : 1, and further preferred of ≥ 0.4 : 1 to ≤ 0.95 : 1 and in addition preferred of ≥ 0.5 : 1 to ≤ 0.95 : 1.

The disinfectant cleaner composition of the invention can be used for inactivating and/or reducing infectious agents of mycobacteria, and/or Adenovirus and Simianvirus 40, comprising bacteria, virus, and/or yeasts, on hard and/or soft surfaces, preferably for inactivating tuberculosis causing organism or other pathogens from the group of mycobacteria.

In particular, the disinfectant cleaner composition of the invention can be used for activating and/or reducing virus on hard and/or soft surfaces that are mycobacteria, and/or Adenovirus and Simianvirus 40.

### Definition of terms

So that the invention maybe more readily understood, certain terms are first defined.

The disinfectant cleaner composition of the invention having mycobactericidal effectivity according to EN 14348 and/or in a surface test according to DGHM guideline 2001.

As used herein, "weight percent", "wt-%", "percent by weight", "% by weight", and variations thereof refer to a composition, component, substance or agent as the weight of that composition, component, substance or agent of the disinfectant cleaner composition divided by the total weight of the disinfectant cleaner composition or use composition and multiplied by 100. It is understood that the total weight percent amount of all components, substances or agents of the disinfectant cleaner composition as well as use composition are selected such that it does not exceed 100 wt.-%.

It is understood that, as used here, "percent", "%", are intended to be synonymous with "weight percent", "wt-%".

It should be noted that, as used in this specification and the appended claims, the term "N-dodecyl dimethyl benzyl ammonium chloride" stands for N-dodecyl dimethyl benzyl ammonium chloride or a mixture of quaternary ammonium chloride comprising N-alkyl dimethyl benzyl ammonium with C12 to C18 alkyl containing N-dodecyl dimethyl benzyl ammonium chloride as the main component of at least > 50% wt.-%, based on the total amount of N-alkyl dimethyl benzyl ammonium with C12 to C18 alkyl.

It should also be noted that the term "or" is generally employed in its sense including "and/or" unless the content clearly dictates otherwise.

### Disinfectant composition

The disinfectant cleaner composition comprises phenoxy ethanol.

It has surprisingly been found that the tuberculocidal efficacy and efficacy against Norovirus, Adenovirus and Polyomavirus can be further increased by the addition of phenoxy ethanol to the disinfectant composition.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxyethanol; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect of the invention, the disinfectant cleaner composition comprises in addition at least one alkali source, preferably selected from the group comprising alkali metal hydroxides, alkali metal salts and/or amines and mixtures thereof, preferably triethanol amine, sodium hydroxide, potassium hydroxide, sodium carbonate, and/or sodium bicarbonate and mixtures thereof and more preferred ethanolamine.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxy ethanol;
d) at least one alkali source; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect of the invention, the disinfectant cleaner composition comprises in addition at least one nonionic surfactant. The surfactant may increase the penetration of the active components thus has a beneficial effect of the disinfectant efficacy.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxy ethanol;
d) optional at least one alkali source;
e) at least one nonionic surfactant; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect of the invention, the disinfectant cleaner composition comprises in addition at least one corrosion inhibitor. The disinfection composition is a mild composition and possesses at the most a low corrosive potential with respect to metal surfaces, such as metal surgical instruments. In order to minimize the corrosive potential of the disinfection composition of the invention to a minimum at least one corrosion inhibitor can be added.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxy ethanol;
d) optional at least one alkali source;
e) optional at least one nonionic surfactant;
f) at least one corrosion inhibitor; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect of the invention, the disinfectant cleaner composition comprises in addition at least one sequestering agent. The sequestering agents may remove contaminants, such as iron, manganese, calcium and minimizing color, scale, deposits and/or corrosion.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxy ethanol;
d) optional at least one alkali source;
e) optional at least one nonionic surfactant;
f) optional at least one corrosion inhibitor;
g) at least one sequestering agent and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect of the invention, the disinfectant cleaner composition may be in form of a liquid composition. Preferably, the disinfectant cleaner composition may be in form of a concentrated liquid composition. However, the disinfectant cleaner composition may be in form of a diluted liquid composition, that can be directly applied on the surface to be disinfected, also referred to as "ready-to-use" solution or "ready-to-use" composition.

According to one embodiment of the invention, the disinfectant cleaner composition comprises:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) phenoxy ethanol;
d) optional at least one alkali source;
e) optional at least one nonionic surfactant;
f) optional at least one corrosion inhibitor;
g) optional at least one sequestering agent:
h) at least one solvent; and wherein
the weight ratio of a a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

### Components used in a disinfectant cleaner composition of the Invention

The preferred compositions of the invention comprise ingredients, which are cooperative in their combined effects and effective in inactivating and/or reducing infectious agents of mycobacteria, and/or Adenovirus and Simianvirus 40. Thus, the disinfectant cleaner composition of the invention has for example mycobacteria, and/or Adenovirus and Simianvirus 40 effectivity.

The disinfectant cleaner composition of the invention can be free of nonionic tensides, cationic tensides except N-alkyl dimethyl benzyl ammonium chloride and/or amphoteric tensides.

The disinfectant cleaner composition of the invention can be free of a nonionic surfactant coupler and/or alkoxylated nonionic surfactant, or both.

It should be understood that the disinfectant cleaner composition of the invention can be free of a hydrotrope component.

It should be understood that the disinfectant cleaner composition of the invention can be free of a phosphate.

It should be understood that the disinfectant cleaner composition of the invention can be free of at least one additive, preferably all additives, selected from the group fragrances, dyes, antistatic agents, UV absorbers, reducing agents and/or buffering compounds.

### N-alkyl dimethyl benzyl ammonium chloride

The disinfectant cleaner composition of the invention comprises at least one N-alkyl dimethyl benzyl ammonium chloride. The N-alkyl dimethyl benzyl ammonium chloride, wherein said alkyl has from 8 to 18 carbon atoms, also known as benzalkonium chloride and ADBAC, is a cationic surface-acting agent belonging to the quaternary ammonium group. It has three main categories of use: as a biocide, a cationic surfactant, and phase transfer agent in the chemical industry.

The least one quaternary ammonium chloride can be:
at least one N-alkyl dimethyl benzyl ammonium chloride, wherein said alkyl has from 8 to 18 carbon atoms, can be selected from the group of at least one alkyl(C14) dimethyl benzyl ammonium chloride, alkyl(C16) dimethyl benzyl ammonium chloride, alkyl(C18) dimethyl benzyl ammonium chloride, a mixture ofN-alkyl(C8-C18) dimethyl benzyl ammonium chloride, or a mixture of N-alkyl(C10-C18) dimethyl benzyl ammonium chloride, or any combination thereof; and more preferred a N-alkyl(C10-C18) dimethyl benzyl ammonium chloride mixture, further further preferred dodecyl dimethyl benzyl ammonium chloride and most preferred a N-alkyl(C10-C18) dimethyl benzyl ammonium chloride mixture, wherein the weight amount, based on the N-alkyl(C10-C18)dimethyl benzyl ammonium chloride mixture, of the N-dodecyl dimethyl benzyl ammonium chloride (C12) is > than N-quatrodecyl dimethyl benzyl ammonium chloride (C14) is > than N-hexadecyl dimethyl benzyl ammonium chloride (C16) is > than N-decyl dimethyl benzyl ammonium chloride (C10) > than N-octadecyl dimethyl benzyl ammonium chloride (C18).

A disinfectant cleaner composition of the invention, preferably in form of a concentrate, may comprise ≥ 4 wt.-% to ≤ 11 wt.-%, preferably ≥ 5 wt.-% to ≤ 10 wt.-%, more preferred ≥ 6 wt.-% to ≤ 9 wt.-%, and most preferred of ≥ 7 wt.-% to ≤ 8 wt.-% of:
- at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; based on the total weight amount of the disinfectant cleaner composition of the invention, preferably in form of a concentrate.

A diluted disinfectant cleaner composition of the invention may comprise ≥ 0.005 wt.-% to ≤ 0.6 wt.-%, preferably ≥ 0.01 wt.-% to ≤ 0.5 wt.-%, more preferred ≥ 0.015 wt.-% to ≤ 0.4 wt.-%, and most preferred of ≥ 0.015 wt.-% to ≤ 0.3 wt.-% of:
- at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; based on the total weight amount of the diluted disinfectant cleaner composition of the invention.

### bis(3-aminopropyl) alkylamine

The disinfectant cleaner composition of the invention comprises at least one bis(3-aminopropyl) alkylamine. The bis(3-aminopropyl) alkylamine, wherein said alkyl has from 6 to 18 carbon atoms, is known to be effective against mycobacteria, and/or Adenovirus and Simianvirus 40 in low concentrations. The bis(3-aminopropyl) alkylamine, wherein said alkyl has from 6 to 18 carbon atoms, is not known to have antiviral efficacy against Norovirus, Adenovirus and/or Polyomavirus.

The bis(3-aminopropyl) alkylamine, wherein said alkyl has from 6 to 18 carbon atoms, may be selected from the group comprising a bis(3-aminopropyl) C6-C18-alkylamine, a bis(3-aminopropyl) octylamine, a bis(3-aminopropyl) decylamine, a bis(3-aminopropyl) dodecylamine, a bis(3-aminopropyl) quatrodecylamine, a bis(3-aminopropyl) hexadecylamine, a bis(3-aminopropyl) octadecylamine, or any combination thereof, and most preferred is a bis(3-aminopropyl) dodecylamine.

A disinfectant cleaner composition of the invention, preferably in form of a concentrate, may comprise ≥ 4 wt.-% to ≤ 12 wt.-%, preferably ≥ 5 wt.-% to ≤ 11 wt.-%, more preferred ≥ 6 wt.-% to ≤ 10 wt.-%, and most preferred of ≥ 7 wt.-% to ≤ 9 wt.-% of at least one bis(3-aminopropyl) C8-C18 alkylamine, or any combination thereof, and most preferred bis(3-aminopropyl) dodecylamine; based on the total weight amount of the disinfectant cleaner composition of the invention, preferably in form of a concentrate.

A diluted disinfectant cleaner composition of the invention may comprise ≥ 0.01 wt.-% to ≤ 0.6 wt.-%, preferably ≥ 0.015 wt.-% to ≤ 0.5 wt.-%, more preferred ≥ 0.02 wt.-% to ≤ 0.45 wt.-%, and most preferred of ≥ 0.02 wt.-% to ≤ 0.4 wt.-% of at least one bis(3-aminopropyl) C8-C18 alkylamine, or any combination thereof, and most preferred bis(3-aminopropyl) dodecylamine; based on the total weight amount of the diluted disinfectant cleaner composition of the invention.

### Phenoxy alkanol

The disinfectant cleaner composition of the invention comprises phenoxy ethanol. Phenoxy ethanol is not known to have antiviral efficacy against Norovirus, Adenovirus and/or Polyomavirus.

A disinfectant cleaner composition of the invention, preferably in form of a concentrate, may comprise ≥ 0 wt.-% to ≤ 20 wt.-%, preferably ≥ 5 wt.-% to ≤ 15 wt.-%, more preferred ≥ 8 wt.-% to ≤ 12 wt.-%, and most preferred of ≥ 9 wt.-% to ≤ 11 wt.-% of phenoxy ethanol; based on the total weight amount of the disinfectant cleaner composition of the invention, preferably in form of a concentrate.

A diluted disinfectant cleaner composition of the invention may comprise ≥ 0 wt.-% to ≤ 1 wt.-%, preferably ≥ 0.01 wt.-% to ≤ 0.8 wt.-%, more preferred ≥ 0.02 wt.-% to ≤ 0.6 wt.-%, and most preferred of ≥ 0.02 wt.-% to ≤ 0.5 wt.-% of phenoxy ethanol; based on the total weight amount of the diluted disinfectant cleaner composition of the invention.

### Surfactant

The disinfectant cleaner composition may include nonionic surfactants, cationic surfactants, amphoteric surfactants and mixtures thereof. The surfactant component can be used to reduce surface tension.

As used herein, "surfactant" refers to any agent that lowers the surface tension of a liquid, for example water. Exemplary surfactants which may be suitable for use with the present invention are described below. In one embodiment, surfactants may be selected from the group consisting of nonionic, cationic, amphoteric, zwitterionic, and combinations thereof.

Most preferred, the disinfectant cleaner composition is free of an anionic surfactant.

### Nonionic surfactant

Exemplary nonionic surfactants that can be used in the disinfectant cleaner composition of the invention may be alkoxylated, preferably ethoxylated or ethoxylated and propoxylated, fatty acid alkyl esters preferably containing 1 to 4 carbon atoms in the alkyl chain, more particularly the fatty acid methyl esters.

Further surfactants include ethoxylated long chain fatty acid amides where the fatty acid has 8-20 carbon atoms and the amide group is ethoxylated with 1-20 ethylene oxide units.

A further class of nonionic surfactants, which can be used according to the invention, is that of the alkyl polyglycosides (APG). Suitable alkyl polyglycosides satisfy the general Formula RO(G)z where R is a linear or branched, particularly 2-methyl-branched, saturated or unsaturated aliphatic radical containing 4 to 22 and preferably 6 to 18 carbon atoms and G stands for a glycose unit containing 5 or 6 carbon atoms, preferably glucose. The degree of oligomerization z is a number between 1.0 and 4.0 and preferably between 1.1 and 1.4.

Additionally, non-ionic surfactants derived from the condensation of ethylene oxide with the product resulting from the reaction of propylene oxide and ethylene diamine are also useful. For example, there are compounds containing from 40% to 80% of polyoxyethylene by weight and having a molecular weight from 5,000 to 11,000 resulting from the reaction of ethylene oxide groups with a hydrophobic base constituted of the reaction product from ethylene diamine and excess propylene oxide wherein the base has a molecular weight on order of 2,500-3, 000.

Suitable nonionic surfactants include the polyoxyethylene-polyoxypropylene condensates, which are sold by BASF under the trade name'Pluronic', polyoxyethylene condensates of aliphatic alcohols/ethylene oxide condensates having from 1 to 30 moles of ethylene oxide per mole of coconut alcohol; ethoxylated long chain alcohols sold by Shell Chemical Co. under the trade name 'Neodol', polyoxyethylene condensates of sorbitan fatty acids, alkanolamides, such as the monoalkoanolamides, dialkanolamides and the ethoxylated alkanolamides, for example coconut monoethanolamide, lauric isopropanolamide and lauric diethanolamide; and amine oxides for example dodecyldimethylamine oxide.

Nonionic surfactants that can be used in the composition of the invention include polyalkylene oxide surfactants (also known as polyoxyalkylene surfactants or polyalkylene glycol surfactants). Suitable polyalkylene oxide surfactants include polyoxypropylene surfactants and polyoxyethylene glycol surfactants. Suitable surfactants of this type are synthetic organic polyoxypropylene (PO)-polyoxyethylene (EO) block copolymers. These surfactants include a di-block polymer comprising an EO block and a PO block, a center block of polyoxypropylene units (PO), and having blocks of polyoxyethylene grafted onto the polyoxypropylene unit or a center block of EO with attached PO blocks. Further, this surfactant can have further blocks of either polyoxyethylene or polyoxypropylene in the molecules. A suitable average molecular weight range of useful surfactants can be 1,000 to 40,000 and the weight percent content of ethylene oxide can be 10-80 wt %.

A suitable polyethylene glycol for use in the present invention can have an average mol weight (MW) in the range of ≥ 4000 to ≤ 12000, preferably ≥ 6000 to ≤ 10000 and more preferred of ≥ 7000 to ≤ 8000. Polyethylene glycol that can be used are marketed for example by BASF under the tradename PLURIOL®.

According to the invention, the disinfectant cleaner composition may comprises at least one polyethylene glycol, preferably a polyethylene glycol with an average mol weight in the range of 4.000 to 12.000, and more preferred a polyethylene glycol having an average mol weight of 8,000.

Further exemplary non-ionic surfactants include alkylphenol alkoxylates, and amine oxides such as alkyl dimethylamine oxide or bis(2- hydroxyethyl) alkylamine oxide.

Most preferred, the disinfectant cleaner composition of the invention may comprises at least one nonionic surfactant, preferably at least one C4 to C18 alkyl polyglycoside or any combination thereof, preferably at least one C8 to C16 alkyl polyglycoside and more preferred a mixture of C8 to C16 alkyl polyglycosides.

The disinfectant cleaner composition of the invention, preferably in form of a concentrate, may comprises ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-% of at least one C4 to C18 alkyl polyglycoside or any combination thereof, preferably at least one C6 to C18 alkyl polyglycoside and more preferred a mixture of C8 to C16 alkyl polyglycosides; based on the total weight amount of the disinfectant cleaner composition of the invention, preferably in form of a concentrate.

A diluted disinfectant cleaner composition of the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one C4 to C18 alkyl polyglycoside or any combination thereof, preferably at least one C6 to C18 alkyl polyglycoside and more preferred a mixture of C8 to C16 alkyl polyglycosides; based on the total weight amount of the diluted disinfectant cleaner composition of the invention.

### Cationic surfactans

The disinfectant cleaner composition can contain a cationic surfactant component. The cationic surfactant can be used to provide sanitizing properties. Cationic surfactants that can be used in the disinfectant cleaner composition include, but are not limited to: amines such as primary, secondary and tertiary monoamines with C1-8 alkyl or alkenyl chains, ethoxylated alkylamines, alkoxylates of ethylenediamine, imidazoles such as a 1-(2-hydroxyethyl)-2-imidazoline, a 2-alkyl-1-(2-hydroxyethyl)-2-imidazoline; and poly sulfonate ammonium salts, as for example, alkylpoly sulfonate ammonium chloride surfactants such as n-alkyl(C12-C18)dimethylbenzyl ammonium chloride, n-tetradecyldimethylbenzylammonium chloride monohydrate, and a naphthylene-substituted poly sulfonate ammonium chloride such as dimethyl-1-naphthylmethylammonium chloride. Suitable cationic surfactants include quaternary ammonium compounds having the formula of RR'R"R'''N⁺X⁻, where R, R', R" and R''' are each a C₁-C₂₄ alkyl, aryl or arylalkyl group that can optionally contain one or more P, O, S or N heteroatoms, and X is F, Cl, Br, I or an alkyl sulfate.

Additional preferred cationic surfactants include ethoxylated and/or propoxylated alkyl amines, diamines, or triamines.

Each of R, R', R" and R''' can independently include, individually or in combination, substituents including 6 to 24 carbon atoms, preferably 14 to 24 carbon atoms, and more preferably, 16 to 24 carbon atoms.

Each of R, R', R" and R''' can independently be linear, cyclic, branched, saturated, or unsaturated, and can include heteroatoms such as oxygen, phosphorous, sulfur, or nitrogen. Any two of R, R', R" and R''' can form a cyclic group. Any one of three of R, R', R" and R''' can independently be hydrogen. X is preferably a counter ion and preferably a non-fluoride counter ion. Exemplary counter ions include chloride, bromide, methosulfate, ethosulfate, sulfate, and phosphate.

In an embodiment, the quaternary ammonium compound includes alkyl ethoxylated and/or propoxylated quaternary ammonium salts (or amines).

Preferably, the alkyl group contains between 6 and 22 carbon atoms and can be saturated and/or unsaturated. The degree of ethoxylation is preferably between 2 and 20, and/or the degree of propoxylation is preferably between 0 and 30.

In an embodiment, the quaternary ammonium compound includes an alkyl group with 6 to 22 carbon atoms and a degree of ethoxylation between 2 and 20. A preferred cationic surfactant is commercially available under the name Berol 563 from Akzo-Nobel.

The cationic surfactants can be provided in a disinfectant cleaner composition of the invention, preferably in form of a concentrate, in ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-%; based on the total weight amount of the disinfectant cleaner composition of the invention, preferably in form of a concentrate.

A diluted disinfectant cleaner composition of the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one cationic surfactant; based on the total weight amount of the diluted disinfectant cleaner composition of the invention.

It should be understood that the disinfectant cleaner composition of the invention can be preferably free of a cationic surfactant except for the active components a) of at least one quaternary ammonium chloride of N-alkyl dimethyl benzyl ammonium chloride, wherein said alkyl has from 8 to 18 carbon atoms, further preferred dodecyl dimethyl benzyl ammonium chloride, and b) at least one bis(3-aminopropyl) alkylamine, wherein said alkyl has from 6 to 18 carbon atoms, preferably bis(3-aminopropyl)dodecylamine.

### Amphoteric Surfactants

Amphoteric surfactants can also be used to provide desired detersive properties. Suitable amphoteric surfactants that can be used include, but are not limited to: betaines, imidazolines, and propionates. Suitable amphoteric surfactants include, but are not limited to: sultaines, amphopropionates, amphodipropionates, aminopropionates, aminodipropionates, amphoacetates, amphodiacetates, and amphohydroxypropylsulfonates. When the disinfectant cleaner composition includes an amphoteric surfactant, the amphoteric surfactant can be included in an amount of ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-% of at least one amphoteric surfactant; based on the total weight amount of the disinfectant cleaner composition of the invention, preferably in form of a concentrate.

A diluted disinfectant cleaner composition of the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one amphoteric surfactant; based on the total weight amount of the diluted disinfectant cleaner composition of the invention.

It should be understood that the disinfectant cleaner composition of the invention can be preferably free of an amphoteric surfactant.

### Alkaline Source

The source of alkalinity can be any source of alkalinity that is compatible with the other components of the disinfectant cleaner composition and that will provide the solution with the desired pH.

Exemplary sources of alkalinity include alkali metal hydroxides, alkali metal salts, amines, and mixtures thereof.

Exemplary alkali metal hydroxides include sodium hydroxide, potassium hydroxide, and lithium hydroxide.

Exemplary alkali metal salts include sodium carbonate, trisodium phosphate, potassium carbonate, and mixtures thereof.

Exemplary amines include alkanolamine selected from the group comprising triethanolamine, monoethanolamine, diethanolamine, and mixtures thereof.

The source of alkalinity, preferably an alkali metal hydroxide, may be added to the disinfectant cleaner composition in a variety of forms, including for example in the form of solid beads, dissolved in an aqueous solution or a combination thereof. Alkali metal hydroxides are commercially available as pellets or beads having a mix of particle sizes ranging from 12-100 U. S. mesh, or as an aqueous solution, as for example, as 45 wt. %, 50 wt. % and 73 wt. % solution.

Preferably the alkalinity source is selected from the group comprising alkali metal hydroxides, alkali metal salts, phosphates and/or amines and mixtures thereof, preferably triethanol amine, sodium hydroxide, potassium hydroxide, sodium carbonate, and/or sodium bicarbonate and mixtures thereof and more preferred ethanolamine.

According to the invention, the disinfectant composition, preferably in form of a concentrate, may comprises ≥ 0 wt.-% to ≤ 10 wt.-%, preferably ≥ 0.1 wt.-% to ≤ 8 wt.-%, more preferred ≥ 0.5 wt.-% to ≤ 5 wt.-%, and most preferred of ≥ 1 wt.-% to ≤ 3 wt.-% of at least one alkali source, and more preferred ethanolamine; based on the total weight amount of the disinfectant cleaner composition of the invention, preferably in form of a concentrate.

A diluted disinfectant cleaner composition of the invention may comprise ≥ 0 wt.-% to ≤ 0.5 wt.-%, preferably ≥ 0.00025 wt.-% to ≤ 0.4 wt.-%, more preferred ≥ 0.001 wt.-% to ≤ 0.2 wt.-%, and most preferred of ≥ 0.004 wt.-% to ≤ 0.1 wt.-% of at least one alkali source, and more preferred ethanolamine; based on the total weight amount of the diluted disinfectant cleaner composition of the invention.

It should be understood that the disinfectant cleaner composition of the invention can be preferably free of an alkali source.

It should be understood that the disinfectant cleaner composition of the invention can be preferably free of phosphates.

### Corrosion inhibitor

According to the present invention, at least one corrosion inhibitor maybe used to prevent the corrosion of an article to be cleaned.

Preferably a silicate(s) corrosion inhibitor and more preferred a disilicate corrosion inhibitor can be used in the disinfectant cleaner composition according to the present invention. The silicate(s) and/or disilicate corrosion inhibitor can be an alkali silicate and/or alkali disilicate.

Other inhibitors that can be used can be selected from the group comprising calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole and mixtures thereof.

More preferred, the corrosion inhibitor is a heterocyclic compound, a triazole derivate, such as a benzotriazole or 1,2,3-benzotriazole and mixtures thereof.

Exemplary silicates include sodium metasilicates, sesquisilicates, orthosilicates, potassium silicates, and mixtures thereof. However, most preferred can be sodium silicate.

The silicates may comprise at least one crystalline layer-forming silicate of the general formula NaMSixO2x+1.yH2O, wherein M represents sodium or hydrogen, x is a number from 1.9 to 22, preferably 1.9 to 4 and y stands for a number from 0 to 33.

The crystalline layer-forming silicates of the formula NaMSixO22x+1.yH2O are marketed for example by Clariant GmbH (Germany) under the trade names Na-SKS, eg. Na-SKS-1 (Na2Si22O45.xH2O, Kenyait), Na-SKS-2 (Na2Si14O29.xH2O, Magadiit), Na-SKS-3 (Na2Si8O17.xH2O) or Na-SKS-4 (Na2Si4O9.xH2O, Makatit).

Crystalline, layered silicates of the above formula, in which x stands for 2, are particularly suitable for the purposes of the present invention.

Na-SKS-5 (alpha -Na2Si2O5), Na-SKS-7 (beta -Na2Si2O5, Natrosilit), Na-SKS-9 (NaHSi2O5.H2O), Na-SKS-10 (NaHSi2O5.3H2O, Kanemit), Na-SKS-11 (t-Na2Si2O5) and Na-SKS-13 (NaHSi2O5) are most notably suitable, particularly Na-SKS-6 (delta -Na2Si2O5).

In the context of the present application, silicates can comprise a content by weight of crystalline layered silicates of formula NaMSixO2x+1.yH2O of 0.1 to 20 wt. %, preferably 0.2 to 15 wt. % and particularly 0.4 to 10 wt. %, each based on the total weight of the corrosion inhibitor agent.

Particularly preferred are especially those that have a total silicate content > 0 and below 7 wt.- %, advantageously below 6 wt.- %, preferably below 5 wt.- %, particularly preferably below 4 wt.- %, quite particularly preferably below 3 wt. -% and especially below 2.5 wt.- %, wherein this silicate, based on the total weight of the comprised silicate, is advantageously at least 70 wt.- %, preferably at least 80 wt.- % and especially at least 90 wt.-% of a silicate of the general formula NaMSixO2x+1.yH2O.

However, other corrosion inhibitors can be suitable added to the disinfectant composition of this invention include magnesium and/or zinc ions and Ca(NO₂)₂. Preferably, the metal ions are provided in water-soluble form.

Examples of useful water-soluble forms of magnesium and zinc ions are the water-soluble salts thereof including the chlorides, nitrates and sulfates of the respective metals. If any of the alkalinity providing agents are the alkali metal carbonates, bicarbonates or mixtures of such agents, magnesium oxide can be used to provide the Mg ion. The magnesium oxide is water soluble and is a preferred source of Mg ions.

In order to maintain the dispersibility of the magnesium and/or zinc corrosion inhibitors in aqueous solution, and in the presence of agents which would otherwise cause precipitation of the zinc or magnesium ions, e. g. , carbonates, it might be advantageous to include a carboxylated polymer to the solution.

The useful carboxylated polymer corrosion inhibitors may be generically categorized as water-soluble carboxylic acid polymers such as polyacrylic and polymethacrylic acids or vinyl addition polymers, in addition to the acid-substituted polymers used in the present invention.

Of the vinyl addition polymer corrosion inhibitors contemplated, maleic anhydride copolymers as with vinyl acetate, styrene, ethylene, isobutylene, acrylic acid and vinyl ethers are examples.

The polymers tend to be water-soluble or at least colloidally dispersible in water. The molecular weight of these polymers may vary over a broad range although it is preferred to use polymers having average molecular weights ranging between 1,000 up to 1,000, 000. These polymers have a molecular weight of 100,000 or less and between 1,000 and 10,000.

The polymers or copolymers (either the acid-substituted polymers or other added polymers) may be prepared by either addition or hydrolytic techniques. Thus, maleic anhydride copolymers are prepared by the addition polymerization of maleic anhydride and another comonomer such as styrene.

The low molecular weight acrylic acid polymer corrosion inhibitors may be prepared by addition polymerization of acrylic acid or its salts either with itself or other vinyl comonomers.

Alternatively, such polymers may be prepared by the alkaline hydrolysis of low molecular weight acrylonitrile homopolymers or copolymers.

According to a more preferred embodiment of the present invention the disinfectant cleaner composition may comprises of at least one corrosion inhibitor selected from the group comprising silicate, sodium silicate, sodium disilicate, calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole, or any combination thereof, more preferred at least one benzotriazole, and most preferred at least one 1,2,3-benzotriazole.

According to the invention, the disinfectant composition, preferably in form of a concentrate, may comprises ≥ 0 wt.-% to ≤ 1 wt.-%, preferably ≥ 0.05 wt.-% to ≤ 0.8 wt.-%, more preferred ≥ 0.1 wt.-% to ≤ 0.6 wt.-%, and most preferred of ≥ 0.15 wt.-% to ≤ 0.4 wt.-% of at least one corrosion inhibitor, preferably benzotriazole, and most preferred at least one 1,2,3-benzotriazole; based on the total weight amount of the disinfectant cleaner composition of the invention, preferably in form of a concentrate.

A diluted disinfectant cleaner composition of the invention may comprise ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one corrosion inhibitor, preferably benzotriazole, and most preferred at least one 1,2,3-benzotriazole; based on the total weight amount of the diluted disinfectant cleaner composition of the invention.

It should be understood that the disinfectant cleaner composition of the invention can be preferably free of a corrosion inhibitor.

### Sequestering agent

The disinfectant cleaner composition may in addition comprises at least one sequestering agent selected from the group of sodium gluconate, pentasodium salt of diethylenetriamine pentaacetic acid (DTPA), sodium glucoheptonate, salts of ethylene diamine tetraacetic acid (EDTA), salts of ethylene diamine tetraacetic acid, salts of hydroxyethyl ethylene diamine triacetic acid, salts of hydroxyethyl ethylene diamine triacetic acid, salts of nitrilotriacetic acid, salts of nitrilotriacetic acid (NTA), diethanolglycine sodium salt, ethanoldiglycine disodium salt, salts of hydroxymonocarboxylic acid compounds, salts of hydroxydicarboxylic acid compounds, salts of amine containing carboxylic acids, terasodium N,N-bis(carboxylatomethyl)-L-glutamate (GLDA), hydroxyethylethylene-diaminetriacetate (HEDTA), or any combination thereof, and more preferred methylglycinediacetate (MGDA).

According to the invention, the disinfectant composition, preferably in form of a concentrate, may comprises ≥ 0 wt.-% to ≤ 1 wt.-%, preferably ≥ 0.05 wt.-% to ≤ 0.8 wt.-%, more preferred ≥ 0.07 wt.-% to ≤ 0.6 wt.-%, and most preferred of ≥ 0.1 wt.-% to ≤ 0.5 wt.-% of at least one sequestering agent, preferably methylglycinediacetate (MGDA); based on the total weight amount of the disinfectant cleaner composition of the invention, preferably in form of a concentrate.

A diluted disinfectant cleaner composition of the invention may comprise ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one sequestering agent, preferably methylglycinediacetate (MGDA); based on the total weight amount of the diluted disinfectant cleaner composition of the invention.

It should be understood that the sequestering agent can include mixtures of different sequestering agents.

It should be understood that the disinfectant cleaner composition can be preferably free of a sequestering agent.

### Additional solvents

Suitable additional solvents include, but are not limited to, water, alcohols, ethanol, isopropanol, 2-butoxy ethanol, 1-decanol, benzyl alcohol, glycerin, monoethanolamine, glycols, ethylene glycol, diethylene glycol, propylene glycol, butoxy diglycol, triethylene glycol, tetraethylene glycol, glycerin, propylene glycol, dipropylene glycol, hexylene glycol, glycol ethers, esters, or combinations thereof. Suitable alcohols include, but are not limited to, ethanol, isopropanol, 2-butoxy ethanol, 1-decanol, benzyl alcohol, glycerin, monoethanolamine, or any combination thereof, and preferably the solvent is water.

According to the invention, the disinfectant composition, preferably in form of a concentrate, may comprises ≥ 0 wt.-% to ≤ 92 wt.-%, preferably ≥ 20 wt.-% to ≤ 80 wt.-%, more preferred ≥ 50 wt.-% to ≤ 75 wt.-%, and further more preferred ≥ 55 wt.-% to ≤ 70 wt.-%, preferably the solvent is add. to 100 wt.-%, and most preferably the solvent is water; based on the total weight amount of the disinfectant cleaner composition of the invention, preferably in form of a concentrate.

A diluted disinfectant cleaner composition of the invention may comprise of at least one solvent of ≥ 0 wt.-% to < 100 wt.-%, preferably ≥ 0.05 wt.-% to ≤ 99,5 wt.-%, more preferred ≥ 0.05 wt.-% to ≤ 99 wt.-%, and further more preferred ≥ 0.07 wt.-% to ≤ 99 wt.-%, preferably the solvent is add. to 100 wt.-%, and most preferably the solvent is water; based on the total weight amount of the diluted disinfectant cleaner composition of the invention.

### Concentrate

The disinfectant cleaner composition can be presented in a liquid concentrated form. The source of alkalinity and addition of the solvent, preferably water, are provided so that the concentrated, preferably aqueous, liquid composition of the disinfectant cleaner composition according to the present invention may have a pH in the range of ≥ 7 pH to ≤ 14 pH, preferably is from ≥ 9 pH to ≤ 13 pH, and more preferred is from ≥ 10 pH to ≤ 12 pH.

According to one embodiment, the disinfectant cleaner composition of the invention, preferably in form of a concentrate, may comprise:
- ≥ 4 wt.-% to ≤ 11 wt.-%, preferably ≥ 5 wt.-% to ≤ 10 wt.-%, more preferred ≥ 6 wt.-% to ≤ 9 wt.-%, and most preferred of ≥ 7 wt.-% to ≤ 8 wt.-% of at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; and/or mixtures thereof;
- ≥ 4 wt.-% to ≤ 12 wt.-%, preferably ≥ 5 wt.-% to ≤ 11 wt.-%, more preferred ≥ 6 wt.-% to ≤ 10 wt.-%, and most preferred of ≥ 7 wt.-% to ≤ 9 of at least one bis(3-aminopropyl) C8-C18 alkylamine, or any combination thereof, and most preferred bis(3-aminopropyl) dodecylamine;
- ≥ 5 wt.-% to ≤ 15 wt.-%, more preferred ≥ 8 wt.-% to ≤ 12 wt.-%, and most preferred of ≥ 9 wt.-% to ≤ 11 wt.-% phenoxy ethanol;
- ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 1 wt.-%, preferably ≥ 0.05 wt.-% to ≤ 0.8 wt.-%, more preferred ≥ 0.1 wt.-% to ≤ 0.6 wt.-%, and most preferred of ≥ 0.15 wt.-% to ≤ 0.4 wt.-% of at least one corrosion inhibitor, preferably benzotriazole, and most preferred at least one 1,2,3-benzotriazole;
- ≥ 0 wt.-% to ≤ 1 wt.-%, preferably ≥ 0.05 wt.-% to ≤ 0.8 wt.-%, more preferred ≥ 0.07 wt.-% to ≤ 0.6 wt.-%, and most preferred of ≥ 0.1 wt.-% to ≤ 0.5 wt.-% of at least one sequestering agent, preferably methylglycinediacetate (MGDA);
- ≥ 0 wt.-% to ≤ 10 wt.-%, preferably ≥ 0.1 wt.-% to ≤ 8 wt.-%, more preferred ≥ 0.5 wt.-% to ≤ 5 wt.-%, and most preferred of ≥ 1 wt.-% to ≤ 3 wt.-% of at least one alkali source, and more preferred ethanolamine;
- a solvent of ≥ 0 wt.-% to ≤ 92 wt.-%, preferably ≥ 20 wt.-% to ≤ 80 wt.-%, more preferred ≥ 50 wt.-% to ≤ 75 wt.-%, and further more preferred ≥ 55 wt.-% to ≤ 70 wt.-%, preferably the solvent is add. to 100 wt.-%, and most preferably the solvent is water; wherein
the weight.-% of the components are based on the total weight of disinfectant cleaner composition and the weight.-% of all components of the composition are select so that it does not exceed 100 wt.-%.

### Ready-to-use composition

The disinfectant cleaner composition of the invention can be present in form of a diluted or so called "ready-to-use" composition. The source of alkalinity and addition of the solvent, preferably water, are provided so that the diluted, preferably aqueous, liquid composition of the disinfectant cleaner composition according to the present invention may have a pH in the range of ≥ 7 pH to ≤ 12 pH, preferably is from ≥ 7.5 pH to ≤ 11,5 pH, and more preferred is from ≥ 9 pH to ≤ 11,0 pH.

According to one aspect of the invention, the concentrated disinfectant cleaner composition can be diluted with a at least one solvent, preferably water, by a factor of 10 to 1000, preferably 20 to 500 and further preferred 25 to 400 to obtain the diluted disinfectant cleaner composition of the invention.

According to one aspect, the diluted disinfectant cleaner composition (ready-to-use) can be diluted with a solvent, preferably water, to a 0.25% to 4.0% solution from a concentrated disinfectant composition.

It will be appreciated that the actual concentration of components in a composition of the invention will depend on the intended use of that composition. For disinfecting uses, such as cleaning of hospital wards and equipment to help prevent the spread of disease such as Norovirus, Adenovirus and Polyomavirus, higher concentrations are required than for certain sanitizing applications.

According to one embodiment, the diluted disinfectant cleaner composition may comprise:
- ≥ 0.005 wt.-% to ≤ 0.6 wt.-%, preferably ≥ 0.01 wt.-% to ≤ 0.5 wt.-%, more preferred ≥ 0.015 wt.-% to ≤ 0.4 wt.-%, and most preferred of ≥ 0.015 wt.-% to ≤ 0.3 wt.-% of at least one at least one N-alkyl dimethyl benzyl ammonium chloride, wherein said alkyl has from 8 to 18 carbon atoms, can be selected from the group of at least one alkyl(C14) dimethyl benzyl ammonium chloride, alkyl(C16) dimethyl benzyl ammonium chloride, alkyl(C18) dimethyl benzyl ammonium chloride, a mixture of N-alkyl(C8-C18) dimethyl benzyl ammonium chloride, or a mixture of N-alkyl(C10-C18) dimethyl benzyl ammonium chloride, or any combination thereof; and more preferred a N-alkyl(C10-C18) dimethyl benzyl ammonium chloride mixture, further further preferred dodecyl dimethyl benzyl ammonium chloride and most preferred a N-alkyl(C10-C18) dimethyl benzyl ammonium chloride mixture, wherein the weight amount, based on the N-alkyl(C10-C18)dimethyl benzyl ammonium chloride mixture, of the N-dodecyl dimethyl benzyl ammonium chloride (C12) is > than N-quatrodecyl dimethyl benzyl ammonium chloride (C14) is > than N-hexadecyl dimethyl benzyl ammonium chloride (C16) is > than N-decyl dimethyl benzyl ammonium chloride (C10) > than N-octadecyl dimethyl benzyl ammonium chloride (C18) and/or mixtures thereof;
- ≥ 0.01 wt.-% to ≤ 0.6 wt.-%, preferably ≥ 0.015 wt.-% to ≤ 0.5 wt.-%, more preferred ≥ 0.02 wt.-% to ≤ 0.45 wt.-%, and most preferred of ≥ 0.02 wt.-% to ≤ 0.4 wt.-% of at least one bis(3-aminopropyl) C8-C18 alkylamine, or any combination thereof, and most preferred bis(3-aminopropyl) dodecylamine;
- > 0 wt.-% to ≤ 1 wt.-%, preferably ≥ 0.01 wt.-% to ≤ 0.8 wt.-%, more preferred ≥ 0.02 wt.-% to ≤ 0.6 wt.-%, and most preferred of ≥ 0.02 wt.-% to ≤ 0.5 wt.-% phenoxy ethanol;
- ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one corrosion inhibitor, preferably benzotriazole, and most preferred at least one 1,2,3-benzotriazole;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one sequestering agent, preferably methylglycinediacetate (MGDA);
- ≥ 0 wt.-% to ≤ 0.5 wt.-%, preferably ≥ 0.00025 wt.-% to ≤ 0.4 wt.-%, more preferred ≥ 0.001 wt.-% to ≤ 0.2 wt.-%, and most preferred of ≥ 0.004 wt.-% to ≤ 0.1 wt.-% of at least one alkali source, and more preferred ethanolamine;
- a solvent of ≥ 0 wt.-% to < 100 wt.-%, preferably ≥ 0.05 wt.-% to ≤ 99,5 wt.-%, more preferred ≥ 0.05 wt.-% to ≤ 99 wt.-%, and further more preferred ≥ 0.07 wt.-% to ≤ 99 wt.-%, preferably the solvent is add. to 100 wt.-%, and most preferably the solvent is water; wherein
the weight.-% of the components are based on the total weight of disinfectant cleaner composition and the weight.-% of all components of the composition are select so that it does not exceed 100 wt.-%.

### Use of the disinfectant composition

The disinfectant cleaner composition according to the invention can be used in a method for inactivating and/or reducing infectious agents of mycobacteria, and/or Adenovirus and Simianvirus 40. More specifically, the composition has a mycobactericidal efficacy and efficacy against Adenovirus and Simianvirus 40.

The disinfectant cleaner composition of the invention gives a reduction in the number of microorganisms and viruses, preferably Norovirus, Adenovirus and/or Polyomavirus which is at least log 3.0. Preferably a disinfectant cleaner composition of the invention having a residual effect and tested in this manner will give a log reduction of at least 3.0, more preferably of at least 4.0.

The compositions of the invention inactivate and/or reduce infectious agents of mycobacteria, and/or Adenovirus and Simianvirus 40, for example on surfaces in hospitals, industrial facilities and research laboratories, particularly selected from surfaces of instruments employed in medical, dental, and pharmaceutical procedures, surfaces of equipment, the inanimate patient environment, inanimate surfaces in operating theatres and other areas within healthcare, processing facilities or containers used in the food service, food processing, butchery, dairy, beverage, brewery, and pharmaceutical industries, work surfaces, walls, floors, ceilings, fermentation tanks, and fluid supply lines.

Using the disinfectant compositions according to the invention can take the form of a concentrate that can be diluted and combined to provide a ready-to-use solution, and as a ready-to-use liquid composition that can be used to clean articles having a metal or plastic surface, such as surgical, medical, and dental instruments, including endoscopes.

Metal surfaces and/or plastic surfaces in need of disinfecting and cleaning are found in several locations. Exemplary locations include surgical instruments, medical instruments, and dental instruments, sinks, cookware, utensils, machine parts, vehicles, tanker trucks, vehicle wheels, work surfaces, tanks, immersion vessels, spray washers, and ultrasonic baths.

Metal surfaces that can be disinfected include iron-based metals such as iron, iron alloys, e. g. steel, tin, aluminum, copper, tungsten, titanium, molybdenum, for example. The structure of the metal surface to be disinfected can vary widely. Thus, the metal surface and/or plastic surface can be as a metal and/or plastic part of complex configuration, sheeting, coils, rolls, bars, rods, plates, disks.

More preferred is the use of the disinfectant cleaner composition of the invention, in particular the ready-to-use composition to disinfect metal and/or plastic articles, especially metal instruments, plastic instruments, instruments with a plastic surface and/or instruments with a metal surface, surfaces of equipment, the inanimate patient environment, inanimate surfaces in operating theatres and other areas within healthcare.

The disinfectant composition, preferably the ready-to-use-composition, can be applied to a surface by wiping the treated surface with a saturated cloth, mop, sponge or other suitable delivery mechanism. The composition can also be applied by spraying and/or flooding the surface with the disinfectant composition or by immersion of items in the use solution.

The disinfectant cleaner composition of the invention is maybe suitable for a variety of consumer applications. Examples of the formulations of the invention include, but are not limited to surface cleaners such as those intended for use in bathrooms, kitchens, living areas hard floor cleaners carpet cleaners furniture cleaners, glass/mirror cleaners; toilet care products including solid toilet cleaners such as rim devices and those designed to be placed in the cistern liquid toilet cleaners excluding those comprising hypochlorite bleaches: dishwashing products such as washing up liquids and preparations from dishwashing machines such as dishwashing ≥ 7 pH to ≤ 14 pH, preferably is from ≥ 9 pH to ≤ 13 pH, and more preferred is from ≥ 10 pH to ≤ 12 pH liquids; laundry products such as liquid detergents and fabric conditioners and "2 in 1" products comprising detergent and fabric conditioner; cleaning products intended for use outdoors such as those for cleaning for wood, stone, concrete or plastics, for example patio cleaner, garden furniture cleaners/treatments, BBQ cleaners, wall and fence cleaners/ treatments, plant sprays such as those intended to remove insects such as aphides from plants; food sprays, such as those suitable for use in food preservation; personal care products such as bath and shower products; soaps, including liquid soaps, hand sanitizers, deodorants and antiperspirants, hair care products including shampoos, for example anti-scalp odor shampoos, shampoos for the control of head lice eggs and antidandruff shampoos, hair conditioners, hair styling products such as hair mousses, gels and sprays, skin care products such as shaving products, cosmetics and products for hair removal; baby products including baby cleaning and cleansing products such as baby bath, soaps, wipes, moisturizers, nappy rash cream, products for cleaning surfaces that have regular & high incidence of infant & baby contact; first aid products and products for treating ailments and illnesses, including products for the topical treatment and/or prevention of minor infections such as athletes foot, spot/acne prevention/treatment products; foot hygiene products, including those for use on the foot and those for the toot ware, particularly sports foot wear; products for cleaning and/or deodorizing vehicles such as cars. The invention also provides a process for making the compositions of the invention. The process comprises the steps of mixing at least part of at least one quaternary ammonium chloride of N-alkyl dimethyl benzyl ammonium chloride, wherein said alkyl has from 8 to 18 carbon atoms, further preferred dodecyl dimethyl benzyl ammonium chloride, and adding the at least one bis(3-aminopropyl) alkylamine, wherein said alkyl has from 6 to 18 carbon atoms, preferably bis(3-aminopropyl) dodecylamine and phenoxy ethanol; wherein
the weight-% ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1, in the appropriate amounts to achieve the synergy of the two, and any further components, such as solvent, preferably water. and agitating the resulting mixture until a homogeny solution is formed. Typically, the process to produce the compositions of the invention is carried out at room temperature with stirring. The present invention provides compositions obtainable by the process set out above. The compositions of the invention may be prepared in a concentrated form (i.e. with no solvent or little solvent) and diluted with a solvent, preferably water when used to the diluted disinfectant cleaner solution.

### I. Concentrated disinfectant cleaner composition

**Table 1**

| Concentrated disinfectant cleaner composition | | | | | | | |
|---|---|---|---|---|---|---|---|
| Components wt.-% | C1 | C2 | C3 | V4 | V5 | V6 | V7 |
| Benzalkoniumchlorid*¹ | 22.0 | 7.5 | 22.5 | 7.5 | 7.5 | 7.5 | 7.5 |
| N-(3-aminopropyl)-N-dodecylpropane-1,3-diamine | 0 | 4.0 | 8.0 | 8.0 | 8.0 | 8.0 | 8.0 |
| Phenoxyethanol | 17 | 0 | 0 | 0 | 10 | 10 | 10 |
| Monoethanolamin | 0 | 0 | 0 | 0 | 0 | 2 | 2 |
| MGDA *² | 0 | 0 | 0 | 0 | 0 | 0 | 0.25 |
| Benzotriazol | 0 | 0 | 0 | 0 | 0 | 0 | 0.2 |
| C8 to C16 alkyl polyglycoside | 0 | 0 | 0 | 0 | 0 | 0 | 4 |
| H₂O | add. 100 | add. 100 | add. 100 | add. 100 | add. 100 | add. 100 | add. 100 |

| | | | | | | | |
|---|---|---|---|---|---|---|---|
| *¹ = Alkyl dimethyl benzyl ammonium chloride (C10 1,5%, C12 64,73%, C14 22-29%, C16 2-6%) *² = Methylglycinediacetate (MGDA) | | | | | | | |

### EXAMPLES

### EXAMPLE 1

The concentrated disinfectant cleaner compositions C1, C2, C3, and V4,V5, V6 and V7 were each diluted with water to a 0.5%, 1%, 2%, 3% concentrated diluted disinfectant cleaner composition E1(C1), E2(C2), E3(C3), E4(V4), E5(V5), E6(V6) to E7(V7) respectively.

V5, V6 and V7 as well as E5, E6 and E7 are examples of the invention.

C1, C2, C3 and V4 as well as E1, E2, E3 and E4 are comperative examples.

The European Standard EN 14348 defines the test method (phase 2/ step 1) and the requirements for the mycobacteria activity for disinfectants used in medical area including surface and instruments disinfectants.

The mycobacterial activity is defined as the capability to reduce the viable counts of mycobacteria test strains by a factor of minimum lg 4 within 60 minutes at 20° C under the influence of either clean or dirty conditions.

The tests were run with *Mycobacterium terrae* and *Mycobacterium avium* at 20° C with contact times of 60 and 120 minutes under clean and dirty conditions.

**Table 2**

| Activity against Mycobacterium terrae and Mycobacterium avium | | | | | |
|---|---|---|---|---|---|
| disinfectant cleaner composition | | *Mycobacterium terrae* | | *Mycobacterium avium* | |
| | | clean cond. | dirty cond. | clean cond. | dirty cond. |
| 0.5% | E1 | <1.5 lg / 60 min | <1.87 lg / 60 min | <1.87 lg / 60 min | <1.79 lg/ 60 min |
| 1% | E1 | <1.5 lg / 60 min | <1.87 lg / 60 min | <1.87 lg / 60 min | <1.79 lg/ 60 min |
| 2% | E1 | <1.5 lg / 60 min | <1.87 lg / 60 min | <1.87 lg / 60 min | <1.79 lg/ 60 min |
| 3% | E1 | <1.5 lg / 60 min | <1.87 lg / 60 min | <1.87 lg / 60 min | <1.79 lg/ 60 min |
| 0.5% | E2 | <1.5 lg / 60 min | <1.87 lg / 60 min | <1.87 lg / 60 min | <1.79 lg/ 60 min |
| 1 % | E2 | <1.5 lg / 60 min | <1.87 lg / 60 min | <1.87 lg / 60 min | <1.79 lg/ 60 min |
| 2% | E2 | <1.5 lg / 60 min | <1.87 lg / 60 min | <1.87 lg / 60 min | <1.79 lg/ 60 min |
| 3% | E2 | 2.09 lg / 60 min | 3.0 lg / 60 min | 3.72 lg / 60 min | 2.89 lg / 60 min |
| 0.5% | E3 | <1.5 lg / 60 min | <1.87 lg / 60 min | 2.9 lg / 60 min | 2.0 lg / 60 min |
| 1 % | E3 | <1.5 lg / 60 min | <1.87 lg/ 60 min | 3.55 lg / 60 min | 2.29 lg / 60 min |
| 2% | E3 | <1.5 lg / 60 min | <1.87 lg / 60 min | 3.71 lg / 60 min | 3.99 lg / 60 min |
| 3% | E3 | 2.3 lg / 60 min | <1.87 lg / 60 min | 4.42 lg / 60 min | 4.14 lg / 60 min |
| 0.5% | E4 | | 3.56 lg / 60 min | | >6.54 lg / 60 min |
| | | | 4.53 lg / 120 min | | |
| 1 % | E4 | | 4.98 lg / 60 min | | >6.54 lg / 60 min |
| | | | 6.53 lg / 120 min | | |
| 2% | E4 | | 5.22 lg / 60 min | | >6.54 lg / 60 min |
| | | | 6.32 lg / 120 min | | |
| 3% | E4 | | 5.32 lg / 60 min | | >6.54 lg / 60 min |
| 0.5% | E5 | | 3.82 lg / 60 min | | >6.54 lg / 60 min |
| | | | 6.2 lg / 120 min | | |
| 1 % | E5 | | 4.48 lg / 60 min | | >6.54 lg / 60 min |
| | | | >6.53 lg / 120 min | | |
| 2% | E5 | | 4.78 lg / 60 min | | >6.54 lg / 60 min |
| | | | >6.53 lg / 120 min | | |
| 3% | E5 | | 5.36lg / 60 min | | >6.54 lg / 60 min |
| 0.5% | E6 | | 3.54 lg / 60 min | | >6.54 lg / 60 min |
| | | | >6.53 lg / 120 min | | |
| 1 % | E6 | | 4.72 lg / 60 min | | >6.54 lg / 60 min |
| | | | >6.53 lg / 120 min | | |
| 2% | E6 | | 4.83 lg / 60 min | | >6.54 lg / 60 min |
| | | | >6.53 lg / 120 min | | |
| 3% | E6 | | 5.27 lg / 60 min | | >6.54 lg / 60 min |
| 0.5% | E7 | | 3.66 lg / 60 min | | >6.54 lg / 60 min |
| | | | 6.1 lg / 120 min | | |
| 1 % | E7 | | 4.56 lg / 60 min | | >6.54 lg / 60 min |
| | | | >6.53 lg / 120 min | | |
| 2% | E7 | | 4.93 lg / 60 min | | >6.54 lg / 60 min |
| | | | >6.53 lg / 120 min | | |
| 3% | E7 | | 5.53 lg / 60 min | | >6.54 lg / 60 min |

### EXAMPLE 2

The concentrated disinfectant cleaner compositions C1, C2, C3 and, V4, V5, V6 and V7 were each diluted with water to a 0.5%, 1%, 2%, 3% concentrated diluted disinfectant cleaner composition E1(C1), E2(C2), E3(C3), E4(V4), E5(V5), E6(V6) to E7(V7) respectively.

The guideline of the German Society for the Prevention of Viral Disease (DVV 2008) defines the test method (phase 2/ step 1) and the requirements for the virucidal activity for disinfecttants used in medical area including instruments disinfectants.

The virucidal activity is defined as the capability to reduce the infectivity of test viruses by a factor of minimum lg 4 within the corresponding contact time at 20° C with and without the addition of an organic soil.

The tests were run with Adenovirus and Simianvirus 40 at 20° C with contact times of 60 and 120 minutes with and without organic soil.

**Table 3**

| Activity against Adenovirus and Simianvirus 40 | | | | | |
|---|---|---|---|---|---|
| disinfectant cleaner composition | | *Adenovirus* | | *SV40* | |
| | | Without organic soil | with organic soil | Without organic soil | with organic soil |
| 0.5% | E1 | | | 3.25 lg / 60 min | 2.00 lg / 60 min |
| 1% | E1 | | | 2.63 lg / 60 min | 3.88 lg / 60 min |
| 2% | E1 | | >3.75 lg / 120 min | | |
| 3% | E1 | 2.19 lg / 120 min | | | |
| 0.5% | E2 | | | | |
| 1 % | E2 | | | | |
| 2% | E2 | | | | |
| 3% | E2 | >4.19 lg / 60 min | 2.87 lg / 60 min | >4.38 / 60 min | >4.63 / 60 min |
| 0.5% | E3 | 1.94 lg / 60 min | | >4.25 / 60 min | 2.25 lg / 60 min |
| 1 % | E3 | >4.0 lg / 60 min | | >4.25 / 60 min | >4.63 lg / 60 min |
| 2% | E3 | >4.0 lg / 60 min | >4.19 lg / 60 min | >4.25 / 60 min | >4.63 lg / 60 min |
| 3% | E3 | | | >4.25 / 60 min | >4.63 lg / 60 min |
| 0.5% | E4 | | 1.82 lg / 60 min | | 0.191g / 60 min |
| 1 % | E4 | | 2.0 lg / 60 min | | 3.07 lg / 60 min |
| 2% | E4 | | 3.0 lg / 60 min | | |
| 3% | E4 | | | | |
| 0.5% | E5 | | | | 0.57 lg / 60 min |
| 1 % | E5 | | 2.62 lg / 60 min | | 3.82 lg / 60 min |
| 2% | E5 | | >4.0 lg / 60 min | | >4.63 lg / 60 min |
| 3% | E5 | | | | |
| 0.5% | E6 | | | | 0.78 lg / 60 min |
| 1 % | E6 | | 2.72 lg / 60 min | | 3.98 lg / 60 min |
| 2% | E6 | | >4.0 lg / 60 min | | >4.63 lg / 60 min |
| 3% | E6 | | | | |
| 0.5% | E7 | | | | 0.87 lg / 60 min |
| 1 % | E7 | | 2.88 lg / 60 min | | 3.89 lg / 60 min |
| 2% | E7 | | >4.0 lg / 60 min | | >4.63 lg / 60 min |
| 3% | E7 | | | | |

The results of tables 2 and 3 clear demonstrate the improved mycobacterial activity of E5 to E7 compared to E1 to E3.

## Claims

1. A disinfectant cleaner composition comprising:
a) at least one quaternary ammonium chloride of N-alkyl dimethyl benzyl ammonium chloride, wherein said alkyl has from 8 to 18 carbon atoms;
b) at least one bis(3-aminopropyl) alkylamine, wherein said alkyl has from 6 to 18 carbon atoms;
c) phenoxy ethanol; and
wherein the weight-% ratio of a) a quaternary ammonium chloride of N-alkyl dimethyl benzyl ammonium chloride, wherein said alkyl has from 8 to 18 carbon atoms to b) bis(3-aminopropyl) alkylamine, wherein said alkyl has from 6 to 18 carbon atoms, is in the range of > 0.1 : 1 to < 1 : 1.

2. The disinfectant cleaner composition of claim 1, comprising in addition of at least one alkali source, preferably selected from the group comprising alkali metal hydroxides, alkali metal salts and/or amines and mixtures thereof.

3. The disinfectant cleaner composition of claims 1 or 2, comprising in addition of at least one nonionic surfactant.

4. The disinfectant cleaner composition of claims 1 to 3, comprising in addition of at least one corrosion inhibitor selected from the group comprising silicate, sodium silicate, sodium disilicate, calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole, or any combination thereof.

5. The disinfectant cleaner composition of claims 1 to 4, comprising in addition of at least one sequestering agent selected from the group of sodium gluconate, pentasodium salt of diethylenetriamine pentaacetic acid (DTPA), sodium glucoheptonate, salts of ethylene diamine tetraacetic acid (EDTA), salts of ethylene diamine tetraacetic acid, salts of hydroxyethyl ethylene diamine triacetic acid, salts of hydroxyethyl ethylene diamine triacetic acid, salts of nitrilotriacetic acid, salts of nitrilotriacetic acid (NTA), diethanolglycine sodium salt, ethanoldiglycine disodium salt, salts of hydroxymonocarboxylic acid compounds, salts of hydroxydicarboxylic acid compounds, salts of amine containing carboxylic acids, terasodium N,N-bis(carboxylatomethyl)-L-glutamate (GLDA), hydroxyethylethylene-diaminetriacetate (HEDTA), or any combination thereof.

6. The disinfectant cleaner composition of claims 1 to 5, comprising in addition of at least one solvent selected from the group comprising water, alcohols, ethanol, isopropanol, 2-butoxy ethanol, 1-decanol, benzyl alcohol, glycerin, monoethanolamine, glycols, ethylene glycol, diethylene glycol, propylene glycol, butoxy diglycol, triethylene glycol, tetraethylene glycol, glycerin, propylene glycol, dipropylene glycol, hexylene glycol, glycol ethers, esters, or combinations thereof.

7. The disinfectant cleaner composition of claims 1 to 6, wherein the quaternary ammonium chloride of N-alkyl dimethyl benzyl ammonium chloride is selected from the group of at least one alkyl(C14) dimethyl benzyl ammonium chloride, alkyl(C16) dimethyl benzyl ammonium chloride, alkyl(C18) dimethyl benzyl ammonium chloride, a mixture of N-alkyl(C8-C18) dimethyl benzyl ammonium chloride, or a mixture of N-alkyl(C10-C18) dimethyl benzyl ammonium chloride, or any combination thereof.

8. The disinfectant cleaner composition of claims 1 to 7, wherein the bis(3-aminopropyl) alkylamine is selected from the group comprising a bis(3-aminopropyl) C6-C18-alkylamine, a bis(3-aminopropyl) octylamine, a bis(3-aminopropyl) decylamine, a bis(3-aminopropyl) dodecylamine, a bis(3-aminopropyl) quatrodecylamine, a bis(3-aminopropyl) hexadecylamine, a bis(3-aminopropyl) octadecylamine, or any combination thereof.

9. The disinfectant cleaner composition of claims 1 to 8, wherein the pH of the composition is from ≥ 7 pH to ≤ 14 pH.

10. The disinfectant cleaner composition of claims 1 to 9, in form of a concentrate comprising:
- ≥ 4 wt.-% to ≤ 11 wt.-% of at least one C8 to C18 N-alkyl dimethyl benzyl ammonium chloride, and/or mixtures thereof;
- ≥ 4 wt.-% to ≤ 12 wt.-% of at least one bis(3-aminopropyl) C8-C18 alkylamine, or any combination thereof;
- ≥ 5 wt.-% to ≤ 15 wt.-% of at least one phenoxy ethanol;
- ≥ 0 wt.-% to ≤ 10 wt.-% of at least one C4 to C18 alkyl polyglycoside or any combination thereof;
- ≥ 0 wt.-% to ≤ 1 wt.-% of at least one corrosion inhibitor;
- ≥ 0 wt.-% to ≤ 1 wt.-% of at least one sequestering agent;
- ≥ 0 wt.-% to ≤ 10 wt.-% of at least one alkali source;
- a solvent of ≥ 0 wt.-% to ≤ 92 wt.-%; wherein
the weight.-% of the components are based on the total weight of disinfectant cleaner composition and the weight.-% of all components of the composition are select so that it does not exceed 100 wt.-%.

11. The disinfectant cleaner composition of claims 1 to 10 in form of a diluted composition, wherein the concentrated disinfectant cleaner composition is diluted with a at least one solvent.

12. The disinfectant cleaner of claims 1 to 11 for inactivating and/or reducing infectious agents that are mycobacteria, and/or Adenovirus and Simianvirus 40 on hard and/or soft surfaces.

## Patentansprüche

1. Desinfektionsreinigerzusammensetzung, umfassend:
a) mindestens ein quaternäres Ammoniumchlorid aus N-Alkyldimethylbenzylammoniumchlorid, wobei das Alkyl 8 bis 18 Kohlenstoffatome aufweist;
b) mindestens ein Bis(3-aminopropyl)alkylamin, wobei das Alkyl 6 bis 18 Kohlenstoffatome aufweist;
c) Phenoxyethanol; und
wobei das Gewichtsprozent-Verhältnis von a) einem quaternären Ammoniumchlorid aus N-Alkyldimethylbenzylammoniumchlorid, wobei das Alkyl 8 bis 18 Kohlenstoffatome aufweist, zu b) Bis(3-aminopropyl)alkylamin, wobei das Alkyl 6 bis 18 Kohlenstoffatome aufweist, im Bereich von > 0,1: 1 bis < 1: 1 liegt.

2. Desinfektionsreinigerzusammensetzung nach Anspruch 1, ferner umfassend mindestens eine Alkaliquelle, vorzugsweise ausgewählt aus der Gruppe umfassend Alkalimetallhydroxide, Alkalimetallsalze und/oder Amine und Mischungen davon.

3. Desinfektionsreinigerzusammensetzung nach Anspruch 1 oder 2, ferner umfassend mindestens ein nicht-ionisches Tensid.

4. Desinfektionsreinigerzusammensetzung nach Anspruch 1 bis 3, ferner umfassend mindestens einen Korrosionsinhibitor, ausgewählt aus der Gruppe umfassend Silikat, Natriumsilikat, Natriumdisilikat, Calciumacetat, Calciumchlorid, Calciumgluconat, Calciumphosphat, Calciumborat, Calciumcarbonat, Calciumcitrat, Calciumlactat, Calciumsulfat, Calciumtartrat, Benzotriazol, 1,2,3-Benzotriazol oder eine beliebige Kombination davon.

5. Desinfektionsreinigerzusammensetzung nach Anspruch 1 bis 4, ferner umfassend mindestens ein Maskierungsmittel, ausgewählt aus der Gruppe umfassend Natriumgluconat, Pentanatriumsalz der Diethylentriaminpentaessigsäure (DTPA), Natriumglucoheptonat, Salze der Ethylendiamintetraessigsäure (EDTA), Salze der Ethylendiamintetraessigsäure, Salze der Hydroxyethylethylendiamintriessigsäure, Salze der Hydroxyethylethylendiamintriessigsäure, Salze der Nitrilotriessigsäure, Salze der Nitrilotriessigsäure (NTA), Diethanolglycin-Natriumsalz, Ethanoldiglycin-Dinatriumsalz, Salze von Hydroxymonocarbonsäureverbindungen, Salze von Hydroxydicarbonsäureverbindungen, Salze von carbonsäurehaltigen Aminen, Tetranatrium-N,N-bis(carboxylatomethyl)-L-glutamat (GLDA), Hydroxyethylethylendiamintriacetat (HEDTA) oder eine beliebige Kombination davon.

6. Desinfektionsreinigerzusammensetzung nach Anspruch 1 bis 5, ferner umfassend mindestens ein Lösungsmittel ausgewählt aus der Gruppe umfassend Wasser, Alkohol, Ethanol, Isopropanol, 2-Butoxyethanol, 1-Decanol, Benzylalkohol, Glycerin, Monoethanolamin, Glykole, Ethylenglykol, Diethylenglykol, Propylenglykol, Butoxydiglykol, Triethylenglykol, Tetraethylenglykol, Glycerin, Propylenglykol, Dipropylenglykol, Hexylenglykol, Glykolether, Ester oder Kombinationen davon.

7. Desinfektionsreinigerzusammensetzung nach Anspruch 1 bis 6, wobei das quaternäre Ammoniumchlorid aus N-Alkyldimethylbenzylammoniumchlorid ausgewählt ist aus der Gruppe von mindestens einem Alkyl(C14)dimethylbenzylammoniumchlorid, Alkyl(C16)dimethylbenzylammoniumchlorid, Alkyl{C18)dimethylbenzylammoniumchlorid, einer Mischung von N-Alkyl(C8-C18)dimethylbenzylammoniumchlorid oder einer Mischung von N-Alkyl(C10-C18)dimethylbenzylammoniumchlorid oder einer beliebigen Kombination davon.

8. Desinfektionsreinigerzusammensetzung nach Anspruch 1 bis 7, wobei das Bis(3-aminopropyl)alkylamin ausgewählt ist aus der Gruppe umfassend ein Bis(3-aminopropyl)-C6-C18-alkylamin, ein Bis(3-aminopropyl)octylamin, ein Bis(3-aminopropyl)decylamin, ein Bis(3-aminopropyl)dodecylamin, ein Bis(3-aminopropyl)quatrodecylamin, ein Bis(3-aminopropyl)hexadecylamin, ein Bis(3-aminopropyl)octadecylamin oder eine beliebige Kombination davon.

9. Desinfektionsreinigerzusammensetzung nach Anspruch 1 bis 8, wobei der pH-Wert der Zusammensetzung zwischen ≥ 7 und ≤ 14 liegt.

10. Desinfektionsreinigerzusammensetzung nach Anspruch 1 bis 9, in Form eines Konzentrats, umfassend:
- ≥ 4 Gew.-% bis ≤ 11 Gew.-% von mindestens einem C8- bis C18-N-Alkyldimethylbenzylammoniumchlorid und/oder Mischungen davon;
- ≥ 4 Gew.-% bis ≤ 12 Gew.-% von mindestens einem Bis(3-aminopropyl)-C8-C18-Alkylamin oder einer beliebigen Kombination davon;
- ≥ 5 Gew.-% bis ≤ 15 Gew.-% von mindestens einem Phenoxyethanol;
- ≥ 0 Gew.-% bis ≤ 10 Gew.-% von mindestens einem C4- bis C18-Alkylpolyglycosid oder einer beliebigen Kombination davon;
- ≥ 0 Gew.-% bis ≤ 1 Gew.-% von mindestens einem Korrosionsinhibitor;
- ≥ 0 Gew.-% bis ≤ 1 Gew.-% von mindestens einem Maskierungsmittel;
- ≥ 0 Gew.-% bis ≤ 10 Gew.-% von mindestens einer Alkaliquelle;
- ein Lösungsmittel von ≥ 0 Gew.-% bis ≤ 92 Gew.-%;
wobei die Gew.-% der Komponenten auf das Gesamtgewicht der Desinfektionsreinigerzusammensetzung bezogen sind und die Gew.-% aller Komponenten der Zusammensetzung so gewählt sind, dass sie 100 Gew.-% nicht überschreiten.

11. Desinfektionsreinigerzusammensetzung nach Anspruch 1 bis 10 in Form einer verdünnten Zusammensetzung, wobei die konzentrierte Desinfektionsreinigerzusammensetzung mit mindestens einem Lösungsmittel verdünnt wird.

12. Desinfektionsreinigerzusammensetzung nach Anspruch 1 bis 11 zum Inaktivieren und/oder Verringern von Infektionserregern, die Mykobakterien und/oder Adenovirus und Simian-Virus 40 sind, auf harten und/oder weichen Oberflächen.

## Revendications

1. Composition de nettoyage désinfectante comprenant:
a) au moins un chlorure d'ammonium quaternaire de chlorure de N-alkyldiméthylbenzylammonium, dans lequel ledit alkyle contient 8 à 18 atomes de carbone ;
b) au moins une bis(3-aminopropyl)alkylamine, dans lequel ledit alkyle contient de 6 à 18 atomes de carbone ;
c) du phénoxyéthanol ; et
dans lequel le rapport en pourcentage de poids de a) chlorure d'ammonium quaternaire de chlorure de N-alkyldiméthylbenzylammonium, dans lequel ledit alkyle contient de 8 à 18 atomes de carbone à b) bis(3-aminopropyl)alkylamine, dans lequel ledit alkyle contient de 6 à 18 atomes de carbone, est compris entre > 0,1 : 1 et < 1 : 1.

2. Composition de nettoyage désinfectante selon la revendication 1, comprenant en outre au moins une source d'alcali, choisie de préférence dans le groupe comprenant les hydroxydes de métaux alcalins, les sels de métaux alcalins et/ou les amines et leurs mélanges.

3. Composition de nettoyage désinfectante selon les revendications 1 ou 2, comprenant en outre au moins un tensioactif non ionique.

4. Composition de nettoyage désinfectante selon les revendications 1 à 3, comprenant en outre au moins un inhibiteur de corrosion choisi dans le groupe comprenant le silicate, le silicate de sodium, le disilicate de sodium, l'acétate de calcium, le chlorure de calcium, le gluconate de calcium, le phosphate de calcium, le borate de calcium, le carbonate de calcium, le citrate de calcium, le lactate de calcium, le sulfate de calcium, le tartrate de calcium, le benzotriazole, le 1,2,3-benzotriazole, ou une combinaison quelconque de ceux-ci.

5. Composition de nettoyage désinfectante selon les revendications 1 à 4, comprenant en outre au moins un agent séquestrant choisi dans le groupe du gluconate de sodium, du sel pentasodique de l'acide diéthylènetriaminepentaacétique (DTPA), du glucoheptonate de sodium, des sels de l'acide éthylène diamine tétraacétique (EDTA), des sels de l'acide éthylènediamine tétraacétique, des sels de l'acide hydroxyéthyléthylènediaminetriacétique, des sels de l'acide hydroxyéthyléthylènediamine triacétique, des sels de l'acide nitrilotriacétique, des sels de l'acide nitrilotriacétique (NTA), du sel de sodium de diéthanolglycine, du sel de sodium d'éthanoldiglycine, des sels de composés d'acide hydroxymonocarboxyle, des sels de composés d'acide hydroxydicarboxylique, des sels d'amine contenant des acides carboxyliques, du N,N-bis(carboxylatométhyl)-L-glutamate de terasodium (GLDA), de l'hydroxyéthyléthylène-diaminetriacétate (HEDTA), ou une combinaison quelconque de ceux-ci.

6. Composition de nettoyage désinfectante selon les revendications 1 à 5, comprenant en outre au moins un solvant choisi dans le groupe comprenant l'eau, les alcools, l'éthanol, l'isopropanol, le 2-butoxyéthanol, le 1-décanol, l'alcool benzylique, la glycérine, la monoéthanolamine, les glycols, l'éthylène glycol, le diéthylène glycol, le propylène glycol, le butoxy diglycol, le triéthylène glycol, le tétraéthylène glycol, la glycérine, le propylène glycol, le dipropylène glycol, l'hexylène glycol, les éthers de glycol, les esters ou une combinaison de ceux-ci.

7. Composition de nettoyage désinfectante selon les revendications 1 à 6, dans laquelle le chlorure d'ammonium quaternaire de chlorure de N-alkyldiméthylbenzylammonium est choisi dans le groupe constitué par au moins le chlorure d'alkyle (C14) diméthylbenzylammonium, le chlorure d'alkyle (C16) diméthylbenzylammonium, le chlorure d'alkyle (C18) diméthylbenzylammonium, un mélange de chlorure de N-alkyl (C8 à C18) diméthylbenzylammonium, ou un mélange de chlorure de N-alkyl (C10 à C18) diméthylbenzylammonium, ou une combinaison quelconque de ceux-ci.

8. Composition de nettoyage désinfectante selon les revendications 1 à 7, dans laquelle la bis(3-aminopropyl) alkylamine est choisie dans le groupe comprenant une bis(3-aminopropyl) alkylamine en C6 à C18, une bis(3-aminopropyl) octylamine, une bis(3-aminopropyl) décylamine, une bis(3-l'aminopropyl) dodécylamine, une bis(3-aminopropyl) quatrodécylamine, une bis(3-aminopropyl) hexadécylamine, une bis(3-aminopropyl) octadécylamine, ou une combinaison quelconque de celles-ci.

9. Composition de nettoyage désinfectante selon les revendications 1 à 8, dans laquelle le pH de la composition est compris entre ≥ 7 et ≤ 14 pH.

10. Composition de nettoyage désinfectante selon les revendications 1 à 9, sous forme d'un concentré comprenant :
- ≥ 4 % en poids à ≤ 11 % en poids d'au moins un chlorure de N-alkyldiméthylbenzylammonium C8 à C18, et/ou leurs mélanges ;
- ≥ 4 % en poids à ≤ 12 % en poids d'au moins une bis(3-aminopropyl) alkylamine en C8 à C18, ou une combinaison quelconque de celles-ci ;
- ≥ 5 % en poids à ≤ 15 % en poids d'au moins un éthanol phénoxy ;
- ≥ 0 % en poids à ≤ 10 % en poids d'au moins une polyglycoside d'alkyle en C4 à C18 ou une combinaison quelconque de celles-ci ;
- ≥ 0 % en poids à ≤ 1 % en poids d'au moins un inhibiteur de corrosion ;
- ≥ 0 % en poids à ≤ 1 % en poids d'au moins un agent séquestrant ;
- ≥ 0 % en poids à ≤ 10 % en poids d'au moins une source alcaline ;
- un solvant de ≥ 0 % en poids à ≤ 92 % en poids ;
dans laquelle le pourcentage en poids des composants est basé sur le poids total de la composition de nettoyage désinfectante et le pourcentage en poids de tous les composants de la composition est choisi afin de ne pas dépasser 100 % en poids.

11. Composition de nettoyage désinfectante selon les revendications 1 à 10 sous forme d'une composition diluée, dans laquelle la composition de nettoyage désinfectante concentrée est diluée avec un au moins un solvant.

12. Nettoyant désinfectant selon les revendications 1 à 11 pour inactiver et/ou réduire dles agents infectieux qui sont des mycobactéries, et/ou des adénovirus et des virus simiens 40 sur des surfaces dures et/ou souples.
